# EUROPÄISCHE PATENTSCHRIFT

(11) **EP 1 287 818 B1**
(45) Veröffentlichungstag und Bekanntmachung des Hinweises auf die Patenterteilung: **21.11.2007**
(21) Anmeldenummer: 02018322.4
(22) Anmeldetag: 14.08.2002
(51) Int. Cl.: A61K 9/00

(54) **Verfahren zur antibiotischen Beschichtung von Körpern mit interkonnektierenden Mikrohohlräumen, so beschichtete Körper sowie deren Verwendung**
Methods of antibiotic coating of objects having interconnected microcavities and the uses thereof
Méthode de recouvrement avec antibiotiques pour objet à micropores interconnectés et leurs utilisations

(30) Priorität: 31.08.2001 DE 10142465; 01.02.2002 DE 10204307
(43) Veröffentlichungstag der Anmeldung: 05.03.2003
(73) Patentinhaber: Heraeus Kulzer GmbH, 63450 Hanau (DE)
(72) Erfinder: Vogt, Sebastian F., Dr., 07749 Jena (DE); Schnabelrauch, Matthias, Dr., 07749 Jena (DE); Kühn, Klaus-Dieter, Dr., 35041 Marburg (DE)
(74) Vertreter: Kühn, Hans-Christian

(56) Entgegenhaltungen:
- GB-A- 1 400 464
- GB-A- 1 478 240
- US-A- 4 322 398
- US-A- 5 679 646

## Beschreibung

Die vorliegende Erfindung betrifft ein Verfahren zur antibiotischen Beschichtung von Körpern mit interkonnektierenden Mikrohohlräumen, so beschichtete Körper sowie deren Verwendung.

Diese antibiotisch ausgerüsteten Körper mit interkonnektierenden Mikrohohlräumen sollen als Implantate in der Human- und Veterinärmedizin zur Behandlung von Knochendefekten und ggf. zur Behandlung von Weichteildefekten Verwendung finden. Dabei wird eine kontinuierliche Antibiotika-Freisetzung aus der auf der inneren Oberfläche der interkonnektierenden Mikrohöhlräume befindlichen antibiotischen Beschichtung über einen Zeitraum von mehreren Tagen angestrebt, damit eine mikrobielle Infektion im Bereich des zu behandelnden Knochendefekts und oder Weichteildefektes wirksam verhindert oder bekämpft werden kann.

Knochendefekte treten in der Human- und Veterinärmedizin relativ häufig auf und werden insbesondere durch Knochenfisteln, Trümmerfrakturen und Tumoren verursacht. Bei offenen Trümmerfrakturen werden vielfach zusätzlich Infektionen des Knochengewebes beobachtet. Die Behandlung von Knochendefekten kann durch Auffüllung mit geeigneten Implantaten erfolgen. In den letzten Jahren haben insbesondere poröse Implantate Interesse gefunden, die aufgrund ihrer chemischen Zusammensetzung und ihrer Porosität eine osteokonduktive Wirkung aufweisen und ein Einwachsen des umgebenden Knochengewebes begünstigen. Problematisch ist die Behandlung von Knochendefekten immer dann, wenn zusätzlich mikrobielle Infektionen des Knochengewebes vorhanden sind. Infektionen des Knochengewebes können durch systemische oder lokale Applikation von geeigneten Antibiotika bekämpft werden. Die systemische Anwendung von Antibiotika ist aufgrund der mitunter nicht unbeträchtlichen Toxizität der Antibiotika problematisch. Die lokale Applikation direkt im oder am infizierten Gewebe bietet dagegen den Vorteil, dass hohe lokale Antibiotika-Konzentrationen erreicht werden können unter Vermeidung von schädigenden Antibiotika-Konzentrationen im übrigen Organismus. Durch diese hohen lokalen Antibiotika-Konzentrationen am Ort der bakteriellen Infektion ist eine fast vollständige Abtötung der Mikroorganismen möglich, so dass die bakteriellen Infektionen sehr wirksam behandelt werden. Besonders vorteilhaft ist es, wenn am Ort der bakteriellen Infektionen eine wirksame Antibiotikum-Konzentration über einen Zeitraum von mehreren Tagen bis Wochen aufrechterhalten wird, damit das Antibiotikum möglichst tief in das infizierte Gewebe eindringen kann und dadurch auch schwer zugängliche Keime vernichtet werden. Weichteildefekte mit bakteriellen Infektionen sind in der Human- und Veterinärmedizin ebenfalls häufig zu finden. Zur Behandlung dieser Infektionen ist daher auch die lokale Antibiotika-Behandlung von Interesse.

Bisher fanden in Wasser gering lösliche Salze der Aminoglykosid-Antibiotika relativ wenig Beachtung für die Herstellung von Depotpräparaten und von antibiotisch wirksamen Implantaten. Es sind eine Reihe von geringlöslichen Salzen bekannt. So wurde beim Gentamicin die Darstellung geringlöslicher Salze basierend auf höheren Fettsäuren und Arylalkylcarbonsäuren publiziert (G. M. Luedemann, M. J. Weinstein: Gentamycin and method of production. 16.07.1962, US 3,091,572). Exemplarisch sind dafür Gentamicin-Salze der Laurinsäure, der Stearinsäure, der Palmitinsäure, der Ölsäure, der Phenylbuttersäure, der Naphthalen-1-carbonsäure. Die Synthese der Dodecylsulfate des Gentamicins in wässriger bzw. wässrigmethanolischer Lösung sind von Jurado Soler et al. beschrieben worden (A. Jurado Soler, J. A. Ortiz Hernandez, C. Ciuro Bertran: Neue Gentamicinderivate, Verfahren zur Herstellung derselben und diese enthaltende antibiotisch wirksame Zusammensetzung. 30.09.1974, DE 24 46 640). Diese Salze erwiesen sich jedoch vielfach als unvorteilhaft, weil sie wachsartige, hydrophobe Substanzen darstellen, die eine galenische Verwendung behindern. Jurado Soler et al. fanden, dass Gentamicin-pentakis-dodecylsulfat in Lösungsmitteln wie Methanol, Ethanol und Dimethylsulfoxid löslich ist. Sie setzten das Gentamicln-pentakis-dodecylsulfat zur Herstellung von Injektionspräparaten, Salben und Cremes ein. Weitere Verwendungsmöglichkeiten von Gentamicin-pentakis-dodecylsulfat-Lösungen wurden nicht betrachtet. Festtsäuresalze und aliphatische Sulfate von Gentamicin und von Etamycin wurden aus der freien Base bzw. aus ihren Salzen in Wasser bei 50 bis 80 °C synthetisiert (H. Voege, P. Stadler, H. J. Zeiler, S. Samaan, K. G. Metzger: Schwerlösliche Salze von Aminoglykosiden sowie diese enthaltende Formulierungen mit verzögerter Wirkstoff-Freigabe. 28.12.1982, DE 32 48 328). Diese Antibiotika-Fettsäuresalze sollen als Injektionspräparate geeignet sein. Eine neuere Entwicklung stellen schwerlösliche Aminoglykosid-Flavonoid-Phosphate dar (H. Wahlig, E. Dingeldein, R. Kirchlechner, D. Orth, W. Rogalski: Flavonoid phosphate salts of aminoglycoside antibiotics. 13.10.1986, US 4,617,293). Es werden die Salze der Phosphorsäuremonoester von Derivaten der Hydroxyflavane, Hydroxyflavene, Hydroxyflavanone, Hydroxyflavone und Hydroxyflavylium beschrieben. Besonders bevorzugt sind dabei die Derivate der Flavanone und der Flavone. Diese schwerlöslichen Salze sollen als Depotpräparate Verwendung finden. So werden zum Beispiel diese Salze in Kollagenvliese eingebracht (H. Wahlig, E. Dingeldein, D. Braun: Medicinally useful, shaped mass of collagen resorbable in the body. 22.09.1981, US 4,291,013).

Die Erzeugung von einfachen Antibiotikum-/Antibiotka-Depots in den Porensystemen von porösen Körpern durch Tränken von porösen Körpern mit wässrigen Antibiotika-Lösungen ist allgemeiner Kenntnisstand (R. Reiner, W. Kißing, H. Döring, K. Köster, H. Heide: Implantierbares Pharmaka-Depot. 20.02.1978, DE 2807132). Hierbei kann eine retardierende Wirkstofffreisetzung der in Wasser leicht löslichen Antibiotika durch Adsorptions- und oder durch Diffusionsprozesse erreicht werden, die vom verwendeten Material, dem Porenvolumen und der Porosität abhängt. Daneben ist es auch möglich, in Wasser gering lösliche Antibiotika-Salze in geeigneten organischen Lösungsmitteln zu lösen und mit diesen Lösungen die Formkörper zu tränken. Dadurch entstehen Wirkstoffdepots in den Formkörpern, die eine retardierende Wirkstofffreisetzung zeigen. Ein Beispiel dafür ist die von Cimbollek und Nies beschriebene Methode zur Lösung eines in Wasser gering löslichen Gentamicin-Salzes und deren Verwendung zur Beschichtung (M. Cimbollek, B. Nies: Solvent for a sparingly soluble gentamicin salt. 04.05.1994, US 5,679,646). Diese Gentamicinsalz wurde auf Basis von 3-p-Methoxybezylidene-6-hydroxy-4'-methoxyflavanone-6-phosphat synthetisiert. Von Kurtz wird eine sehr interessantes Verfahren beschrieben, bei dem in Wasser gering lösliche Antibiotika-Salze, die aus Gentamicin oder Polymycin und Penicillin oder Cephalosporin aufgebaut sind, in einem organischen Lösungsmittel gelöst werden und mit diesen Lösungen nicht näher spezifizierte Unterlagen getränkt werden (L. D. Kurtz: Wasserunlösliche biocide Antibiotikasalze. 13.11.1973, DE 23 01 633). Die Penicillin- bzw. Cephalosporinreste bilden die anionische Komponente der Salze und die Aminoglukosid-Reste die kationische Komponente.

Zusammenfassend kann festgestellt werden, dass bisher keine Verfahren bekannt sind, bei denen antibiotische Beschichtungen auf der Oberfläche von interkonnektierenden Mikrohohlräumen aufgebracht werden, die aus in Wasser gering löslichen Salzen des Gentamicins bestehen, welche einen anionischen Rest aus der Gruppe der Alkylsulfate und oder Alkylsulfonate enthalten. Die Schichtbildungseigenschaften von in Wasser gering löslichen Antibiotika-Salzen auf Basis von organischen Sulfaten und Sulfonaten fanden bisher keine Beachtung.

Der vorliegenden Erfindung liegt die Aufgabe zugrunde, verbesserte Körper mit antibiotischer Beschichtung, sowie ein einfaches, kostengünstiges Herstellungsverfahren zur antibiotischen Beschichtung von Körpern mit interkonnektierenden Mikrohohlräumen zu entwickeln. Diese antibiotisch ausgerüsteten, Körper mit interkonnektierenden Mikrohohlräumen sollen als Implantate zur Behandlung von Knochen- und Weichteildefekten in der Human- und Veterinärmedizin Verwendung finden. Mit diesem Verfahren sollen in einfacher Weise, unter Verzicht auf polymere Bindemittel, antibiotische Beschichtungen erzeugt werden, die eine Antibiotika-Freisetzung über einen Zeitraum von mehreren Tagen ermöglichen. Die antibiotische Beschichtung soll auf der inneren Oberfläche von Körpern mit interkonnektierenden Mikrohohlräumen gut haften und darf die innterkonnektierenden Mikrohohlräume nicht verschließen.

Die Aufgabe wird durch die Merkmale der unabhängigen Ansprüche gelöst. Vorteilhafte Ausgestaltungen sind in den Unteransprüchen angegeben.

Der Erfindung liegt der überraschende Befund zugrunde, dass antibiotische Beschichtungen mit retardierender Wirkstofffreisetzung in den Mikrohohlräumen von Körpern mit interkonnektierenden Mikrohohlräumen insbesondere dadurch gebildet werden, dass in die Mikrohohlräume eine Lösung von Gentamicin-pentakis-dodecylsulfat oder von Gentamicin-pentakis-dodecylsulfonat in einem geeigneten organischen Lösungsmittel - beispielsweise aus der Gruppe der Alkohole - durch geeignete Maßnahmen wie Tauchen, Sprühen oder Tropfen, eingebracht wird und nach Entfernen (wie etwa durch Verdampfung oder Verdunstung) des organischen Lösungsmittels eine Schicht aus Gentamicin-pentakis-dodecylsulfat oder von Gentamicin-pentakis-dodecylsulfonat auf der Oberfläche der Mikrohohlräume zurückbleibt. Die Mikrohohlräume können als Poren ausgebildet sein.

Die Körper können organischer oder anorganischer Natur sein oder auch sogenannte Composites aus anorganischem und organischem Material. Sie sind z.B. aus Kollagen, Gelatine, Polyestern, Titan, Titanlegierungen, Edelstahl, Calciumcarbonat, Calciumsulfat, Tricalciumphosphat oder Hydroxylapatit aufgebaut. Unter metallischen Körpern mit interkonnektierenden Mikrohohlräumen werden insbesondere solche verstanden, die an ihrer Oberfläche Mikrohohlräume aufweisen, die miteinander verbunden sind, und es werden auch metallische Körper dazugerechnet, deren Oberfläche durch Sandstrahlen so aufgerauht wurde, dass sie offene, miteinander verbundene Hohlräume aufweisen. Es versteht sich, dass die benutzten Lösungen möglichst homogen sind. Als Lösungsmittel kommen vor allem niedere Alkohole sowie N,N-Dimethylformamid (DMF) oder Dimethylsulfoxid (DMSO) in Frage. Bevorzugte Lösungsmittel sind Methanol oder Ethanol.

Gentamicin-pentakis-dodecylsulfat, Gentamicin-tetrakis-dodecylsulfat Gentamicin-tetrakis-dodecylsulfonat und auch Gentamicin-pentakis-dodecylsulfonat sind nichtkristalline, wachsartige Substanzen, die bei der Abdampfung bzw. bei der Verdunstung des organischen Lösungsmittels einen gewissen Verlauf zeigen und sich dabei als Schicht auf Oberflächen auflagern. Sie haften überraschenderweise gut auf Glas-, Keramik- und Kunststoffoberflächen.

Überraschenderweise lösen sich auch Clindamycin-dodecylsulfat, Clindamycin-dodecylsulfonat, Lincosamin-dodecylsulfat, Lincosamin-dodecylsulfonat in Methanol, Ethanol Dimethylsulfoxid und N,N-Dimethylformamid. Diese Stoffe können somit ohne weiteres den Gantamicin-Lösungen zugefügt werden. Es ist auch möglich Tetracyclin-dodecylsulfat oder Tetracylindodecylsulfonat in den Lösungen einzusetzen. Man kann auch anstelle des Tetracyclindodecylsulfates die Dodecylsulfate und oder die Dodecylsulfonate des Chlortetracyclins, des Oxytetracylins, des Demethylchlortetracyclins, des Methacyclins, des Doxycyclins, des Rolitetracyclins und des Minocyclins verwenden. Es kann auch Coprofloxacindodecylbenzylsulfonat zugesetzt werden. Entsprechend entstehen an den Oberflächen der Mikrohohlräume Beschichtungen enthaltend die Gentamicinkomponenten und mindestens eine der genannten weiteren Antibiotika-Komponenten. Im Sinne der Erfindung ist auch die Herstellung von antibiotischen Schichten nur mit den in Methanol, Ethanol, Dimethylsulfoxid und N,N-Dimethylformamid gelösten Dodecylsulfaten, Dodecylsulfonaten und Dodecylbenzylsulfonaten der aufgeführten Antibiotika ohne eine Gentamicin enthaltende antibiotische Komponente.

Überraschenderweise können in Schichten aus Gentamicin-pentakis-dodecylsulfat oder Gentamicin-tetrakis-dodecylsulfat und Gentamicin-pentakis-dodecylsulfonat oder Gentamicin-tetrakis-dodecylsulfonat andere Antibiotika durch Einschluss oder Überdeckung mechanisch fixiert werden. Es ist daher möglich, dass in interkonnektierende Mikrohohlräume von Körpern zuerst eine wässrige Lösung, die mindestens eine in Wasser leicht lösliche antibiotische Komponente aus den Gruppen der Aminoglykosid-Antibiotika, der Tetracyclin-Antibiotika, der Lincosamid-Antibiotika und der 4-Chinolon-Antibiotika enthält, und anschließend nach Verdampfung und oder Verdunstung des Wassers eine Lösung, die aus Gentamicin-pentakis-dodecylsulfat und oder Gentamicin-tetrakis-dodecylsulfat und oder Gentamicin-pentakis-dodecylsulfonat und oder Gentamicin-tetrakis-dodecylsulfonat und dem Lösungsmittel Methanol oder Ethanol oder Dimethylsulfoxid oder N,N-Dimethylformamid besteht, durch Tauchen oder Sprühen oder Tropfen eingebracht wird. Es entsteht im Ergebnis eine Doppelschicht. Bei der Anwendung in Implantaten wird dann das zweite Antibiotikum erst freigesetzt, wenn die Gentamicin-Schicht zumindest teilweise gelöst ist. Als in Wasser leicht lösliche antibiotische Komponente werden in dieser Ausführungsform der Erfindung Gentamicinsulfat, Clindamycinhydrochlorid, Clindamycindihydrogenphosphat, Lincosaminhydrochlorid, Kanamycinsulfat, Amikacinsulfat, Tobramycinsulfat, Tetracyclinhydrochlorid, Chlortetracylinhydrochlorid, Oxytetracyclinhydrochlorid, Demethylchlortetracyclin-hydrochlorid, Methacyclinhydrochlorid, Doxycyclinhydrochlorid, Rolitetracyclinhydrochlorid, Minocyclinhydrochlorid und oder Ciprofloxacinhydrochlorid und oder Moxifloxacinhydrochlorid, bevorzugt eingesetzt.

Die Erfindung betrifft auch ein Verfahren zur antibiotischen Beschichtung von Körpern mit interkonnektierenden Mikrohohlräumen, bei dem in die Mikrohohlräume eine oder mehrere der Substanzen aus der Gruppe Ciprofloxacindodecylbenzylsulfonat und/oder Moxifloxacindodecylsulfat und/oder Moxifloxacindodecylbenzylsulfonat und/oder Moxifloxacindodecylsulfonat und/oder der Dodecylsulfate und/oder der Dodecylsulfonate des Clindamycins, des Tetracyclins, des Lincosamins, des Chlortetracyclins, des Oxytetracylins, des Demethylchlortetracyclins, des Methacyclins, des Doxycyclins, des Rolitetracyclins und des Minocyclins enthaltende Lösung eingebracht wird und nach Verdampfung oder Verdunstung des Lösungsmittels eine Schicht dieser Substanzen auf der Oberfläche der Mikrohohlräume entsteht.

Erfindungsgemäß ist ferner, dass bevorzugt Vliese, Filze, Gewirke oder Gestricke aus Polyestern, Kollagen und Gelatine beschichtet werden.

Das jeweilige Dodecylsulfat oder -sulfonat wird bevorzugt in einer Konzentration von 0,1 bis 20,0 Masseprozent, bezogen auf das Lösungsmittel verwendet.

Erfindungsgemäß ist auch, dass bevorzugt poröse Formkörper aus Polyestern, Calciumcarbonat, Calciumsulfat, Tricalciumphosphat, Hydroxylapatit und resorbierbaren Glas beschichtet werden.

Im Sinne der Erfindung ist, dass die antibiotisch beschichteten Körper mit interkonnektierenden Mikrohohlräumen als Implantate verwendet werden.

Die nachfolgenden Beispiele erklären die Erfindung ohne sie einzuschränken.

### Beispiele

Die Erfindung soll durch die nachstehenden Beispiele 1 und 2 erläutert werden.

Als Körper mit interkonnektierenden Mikrohohlräumen wurden quaderförmige, resorbierbare Phosphatgläser mit den Abmessungen von 20 x 20 x 10 mm für die Beispiele 1 und 2 verwendet. Sie hatten eine Gesamt-Porosität von 65 Volumenprozent.

### Präparation der Beispiele 1 und 2 :

Für die Beispiele kam Gentamicin-pentakis-dodecylsulfat zur Anwendung, dessen Herstellung entsprechend der Vorschrift von Jurado Soler et al. (A. Jurado Soler, J. A. Ortiz Hernandez, C. Ciuro Bertran: Neue Gentamicinderivate, Verfahren zur Herstellung derselben und diese enthaltende antibiotisch wirksame Zusammensetzung. 30.09.1974 DE 24 46 640) erfolgte. Es wurden 135 mg bzw. 270 mg Gentamicin-pentakis-dodecylsulfat in 1 g Methanol gelöst. In die Poren jeweils eines quaderförmigen Phosphatglases wurde die zuvor hergestellte methanolische Lösung getropft. Die Probekörper saugten die Lösung auf und wurden anschließend bei Raumtemperatur bis zur Massekonstanz getrocknet.

**Tab. 1: Zusammensetzungen der verwendeten Lösungen sowie Auswaagen der unbeschichteten und beschichteten Probekörper der Beispiele 1 und 2.**

| Beispiel Nr. | Zusammensetzung der Lösung | Masse der Probekörper vor Beschichtung [mg] | Masse der Probekörper nach Beschichtung [mg] | Masse der Beschichtung [mg] |
|---|---|---|---|---|
| 1 | 135 mg GPDS 1000 mg Methanol | 3949 | 4087 | 130 |
| 2 | 270 mg GPDS 1000 mg Methanol | 3992 | 4257 | 265 |
| GPDS: Gentamicin-pentakis-dodecylsulfat | | | | |

### Antibiotika-Freisetzung der Probekörper der Beispiele 1 und 2:

Die in den Beispielen 1 und 2 hergestellten Formkörper wurden in jeweils 20 ml physiologische Kochsalzlösung eingebracht und in dieser bei 37 °C über einen Zeitraum von 28 Tagen gelagert. Die Probennahme erfolgte nach 1, 2, 3, 6, 9, 13, 15, 21 und 28 Tagen Lagerungszeit. Nach jeder Probennahme wurde das Freisetzungsmedium vollständig durch frisches Medium, ersetzt. Die Antibiotika-Wertbestimmung wurde mit einem Agardiffusionstest unter Verwendung von Bacillus subtilis ATCC 6633 als Testkeim durchgeführt. Die Ergebnisse sind in Tab. 2 dargestellt.

**Tab. 2: Ergebnisse der mikrobiellen Bestimmung der Gentamicin-Freisetzung der beschichteten Probekörper der Beispiele 1 und 2 in Abhängigkeit von der Lagerungszeit der Probekörper in physiologischer Kochsalzlösung bei 37 °C.**

| Beispiel Nr. | Gentamicin-Freisetzung (kumuliert, als Gentamicinsulfat AK=628) [mg] | | | | | | | | |
|---|---|---|---|---|---|---|---|---|---|
| | Freisetzungszeit [d] | | | | | | | | |
| | 1 | 2 | 3 | 6 | 9 | 13 | 15 | 21 | 28 |
| 1 | 1,2 | 1,9 | 2,7 | 3,8 | 4,9 | 5,8 | 6,9 | 8,1 | 9,2 |
| 2 | 1,5 | 3,1 | 4,3 | 5,4 | 6,7 | 7,9 | 8,8 | 10,1 | 11,4 |

## Patentansprüche

1. Verfahren zur antibiotischen Beschichtung von Körpern mit interkonnektierenden Mikrohohlräumen, **dadurch gekennzeichnet, dass** in die Mikrohohlräume eine Gentamicin-dodecylsulfat mit 1 bis 5 Dodecylsulfatgruppen pro Gentamicinmolekül und/oder Gentamicin-dodecylsulfonat mit 1 bis 5 Dodecylsulfonatgruppen pro Gentamicinmolekül enthaltende Lösung eingebracht wird und nach Verdampfung oder Verdunstung des Lösungsmittels eine Schicht des Gentamicin-dodecylsulfat oder Gentamicin-dodecylsulfonat auf der Oberfläche der Mikrohohlräume entsteht - wobei als Lösungsmittel mindestens ein organisches Lösungsmittel eingesetzt wird.

2. Verfahren nach Anspruch 1, **dadurch gekennzeichnet, dass** Gentamicin-pentakis-dodecylsulfat und/oder Gentamicin-tetrakis-dodecylsulfat und/oder Gentamicin-pentakis-dodecylsulfonat und/oder Gentamicin-tetrakis-dodecylsulfonat verwendet wird.

3. Verfahren nach Anspruch 1, **dadurch gekennzeichnet, dass** eines der Lösungsmittel Methanol, Ethanol, N,N-Dimethylformamid und Dimethylsulfoxid eingesetzt wird.

4. Verfahren nach einem der Ansprüche 1 bis 3, **dadurch gekennzeichnet, dass** das Einbringen durch Tauchen oder Sprühen oder Tropfen der Lösung erfolgt.

5. Verfahren nach einem der Ansprüche 1 bis 4, **dadurch gekennzeichnet, dass** die Körper aus Kollagen, Gelatine oder Polyester, Calciumcarbonat, Calciumsulfat, Tricalciumphosphat oder Hydroxylapatit aufgebaut sind.

6. Verfahren nach einem der Ansprüche 1 bis 5, **dadurch gekennzeichnet, dass** zusätzlich eine oder mehrere der Substanzen aus der Gruppe Ciprofloxacindodecylbenzylsulfonat und/oder der Dodecylsulfate und/oder der Dodecylsulfonate des Clindamycins, des Tetracyclins, des Lincosamins, des Chlortetracyclins, des Oxytetracylins, des Demethylchlortetracyclins, des Methacyclins, des Doxycyclins, des Rolitetracyclins und des Minocyclins in der Lösung eingesetzt werden.

7. Verfahren nach einem der Ansprüche 1 bis 6, **dadurch gekennzeichnet, dass** in die interkonnektierenden Mikrohohlräume zuerst eine wässrige Lösung, die mindestens eine in Wasser leicht lösliche antibiotische Komponente aus den Gruppen der Aminoglykosid-Antibiotika, der Tetracyclin-Antibiotika, der Lincosamid-Antibiotika und der 4-Chinolon-Antibiotika enthält, und anschließend nach Verdampfung und/oder Verdunstung des Wassers eine Lösung von Gentamicin-dodecylsulfat oder Gentamicin-dodecylsulfonat, insbesondere von Gentamicin-pentakis-dodecylsulfat und/oder Gentamicin-tetrakis-dodecylsulfat und/oder Gentamicin-pentakis-dodecylsulfonat und/oder Gentamicin-tetrakis-dodecylsulfonat in Methanol, Ethanol, N,N-Dimethylformamid und/oder Dimethylsulfoxid durch Tauchen oder Sprühen oder Tropfen eingebracht wird.

8. Verfahren nach Anspruch 7, **dadurch gekennzeichnet, dass** Gentamicinsulfat, Clindamycinhydrochlorid, Clindamycindihydrogenphosphat, Lincosaminhydrochlorid, Kanamycinsulfat, Amikacinsulfat, Tobramycinsulfat, Tetracyclinhydrochlorid, Chlortetracylinhydrochlorid, Oxytetracyclinhydrochlorid, Demethylchlortetracyclin-hydrochlorid, Methacyclinhydrochlorid, Doxycyclinhydrochlorid, Rolitetracyclinhydrochlorid, Minocyclinhydrochlorid und/oder Ciprofloxacinhydrochlorid und/oder Moxifloxacinhydrochlorid, als in Wasser leicht lösliche antibiotische Komponente(n) eingesetzt werden.

9. Verfahren nach einem der Ansprüche 1 bis 8, **dadurch gekennzeichnet, dass** 0,1 bis 20,0 Masseprozent des Dodecylsulfats oder -sulfonats bezogen auf das Lösungsmittel eingesetzt werden.

10. Verfahren nach einem der Ansprüche 1 bis 9, **dadurch gekennzeichnet, dass** Vliese, Filze, Gewirke oder Gestricke aus Polyester, Kollagen oder Gelatine antibiotisch beschichtet werden.

11. Verfahren nach einem der Ansprüche 1 bis 9, **dadurch gekennzeichnet, dass** poröse Formkörper aus Polyestern, Calciumcarbonat, Calciumsulfat, Tricalciumphosphat, Hydroxylapatit oder resorbierbarem Glas beschichtet werden.

12. Verfahren nach einem der Ansprüche 1 bis 9, **dadurch gekennzeichnet, dass** metallische Formkörper aus Titan, Titanlegierungen oder Edelstahl beschichtet werden.

13. Verfahren zur antibiotischen Beschichtung von Körpern mit interkonnektierenden Mikrohohlräumen, **dadurch gekennzeichnet, dass** in die Mikrohohlräume eine oder mehrere der Substanzen aus der Gruppe Ciprofloxacindodecylbenzylsulfonat und/oder Moxifloxacindodecylsulfat und/oder Moxifloxacindodecylbenzylsulfonat und/oder Moxifloxacindodecylsulfonat und/oder der Dodecylsulfate und/oder der Dodecylsulfonate des Clindamycins, des Tetracyclins, des Lincosamins, des Chlortetracyclins, des Oxytetracylins, des Demethylchlortetracyclins, des Methacyclins, des Doxycyclins, des Rolitetracyclins und des Minocyclins enthaltende Lösung eingebracht wird und nach Verdampfung oder Verdunstung des Lösungsmittels eine Schicht dieser Substanzen auf der Oberfläche der Mikrohohlräume entsteht - wobei als Lösungsmittel mindestens ein organisches Lösungsmittel eingesetzt wird

14. Verfahren nach einem der Ansprüche 1 bis 13, **dadurch gekennzeichnet, dass** die Mikrohohlräume als Poren ausgebildet sind.

15. Körper mit interkonnektierenden Mikrohohlräumen, **dadurch gekennzeichnet, dass** auf der Oberfläche der Mikrohohlräume eine Schicht gebildet ist, die Gentamicin-dodecylsulfat mit 1 bis 5 Dodecylsulfatgruppen pro Gentamicinmolekül oder Gentamicin-dodecylsulfonat mit 1 bis 5 Dodecylsulfonatgruppen pro Gentamicinmolekül aufweist.

16. Körper nach Anspruch 15, **dadurch gekennzeichnet, dass** die Schicht Gentamicin-pentakis-dodecylsulfat und/oder Gentamicin-tetrakis-dodecylsulfat oder Gentamicin-pentakis-dodecylsulfonat und/oder Gentamicin-tetrakis-dodecylsulfonat aufweist.

17. Körper mit interkonnektierenden Mikrohohlräumen, **dadurch gekennzeichnet, dass** auf der Oberfläche der Mikrohohlräume eine Schicht gebildet ist, die eine oder mehrere der Substanzen aus der Gruppe Ciprofloxacindodecylbenzylsulfonat und/oder der Dodecylsulfate und/oder der Dodecylsulfonate des Clindamycins, des Tetracyclins, des Lincosamins, des Chlortetracyclins, des Oxytetracylins, des Demethylchlortetracyclins, des Methacyclins, des Doxycyclins, des Rolitetracyclins und des Minocyclins aufweist.

18. Körper nach Anspruch 15 oder 17, **dadurch gekennzeichnet, dass** er aus Kollagen, Gelatine oder Polyester, Calciumcarbonat, Calciumsulfat, Tricalciumphosphat oder Hydroxylapatit gebildet ist.

19. Körper nach Anspruch 15 oder 17, **dadurch gekennzeichnet, dass** er als Vlies, Filz, Gewirk oder Gestrick aus Polyester, Kollagen oder Gelatine ausgebildet ist.

20. Körper nach Anspruch 17 oder 17, **dadurch gekennzeichnet, dass** er als poröser Formkörper aus Polyester, Calciumcarbonat, Calciumsulfat, Tricalciumphosphat, Hydroxylapatit oder resorbierbarem Glas ausgebildet ist.

21. Körper nach Anspruch 15 oder 17, **dadurch gekennzeichnet, dass** er aus Titan, Titanlegierungen oder Edelstahl ausgebildet ist.

22. Körper nach Anspruch 15 oder 17, **dadurch gekennzeichnet, dass** die Mikrohohlräume als Poren ausgebildet sind.

23. Verwendung von Körpern mit interkonnektierenden Mikrohohlräumen nach einem der Ansprüche 15 bis 22 zur Herstellung von Implantaten.

## Claims

1. Process for the antibiotic coating of bodies with interconnecting microcavities, **characterized in that** a solution containing gentamicin dodecylsulphate having 1 to 5 dodecylsulphate groups per gentamicin molecule and/or gentamicin dodecylsulphonate having 1 to 5 dodecylsulphonate groups per gentamicin molecule is introduced into the microcavities and, after vaporization or evaporation of the solvent, a layer of gentamicin dodecylsulphate or gentamicin dodecylsulphonate forms on the surface of the microcavities - the solvent used being at least one organic solvent.

2. Process according to Claim 1, **characterized in that** gentamicin pentakisdodecylsulphate and/or gentamicin tetrakisdodecylsulphate and/or gentamicin pentakisdodecylsulphonate and/or gentamicin tetrakisdodecylsulphonate is used.

3. Process according to Claim 1, **characterized in that** one of the solvents is methanol, ethanol, N,N-dimethylformamide and dimethyl sulphoxide.

4. Process according to any of Claims 1 to 3, **characterized in that** the introduction is effected by immersion or spraying or dripping of the solution.

5. Process according to any of Claims 1 to 4, **characterized in that** the bodies are composed of collagen, gelatine or polyester, calcium carbonate, calcium sulphate, tricalcium phosphate or hydroxylapatite.

6. Process according to any of Claims 1 to 5, **characterized in that** one or more of the substances from the group consisting of ciprofloxacin dodecylbenzylsulphonate and/or of the dodecylsulphates and/or of the dodecylsulphonates of clindamycin, of tetracycline, of lincosamine, of chlorotetracycline, of oxytetracycline, of demethylchlorotetracycline, of methacycline, of doxycycline, of rolitetracycline and of minocycline are additionally used in the solution.

7. Process according to any of Claims 1 to 6, **characterized in that** first an aqueous solution which contains at least one freely water-soluble antibiotic component from the group consisting of the aminoglycoside antibiotics, of the tetracycline antibiotics, of the lincosamide antibiotics and of the 4-quinolone antibiotics and then, after vaporization and/or evaporation of the water, a solution of gentamicin dodecylsulphate or gentamicin dodecylsulphonate, in particular of gentamicin pentakisdodecylsulphate and/or gentamicin tetrakisdodecylsulphate and/or gentamicin pentakisdodecylsulphonate and/or gentamicin tetrakisdodecylsulphonate, in methanol, ethanol, N,N-dimethylformamide and/or dimethyl sulphoxide is introduced into the interconnecting microcavities by immersion or spraying or dripping.

8. Process according to Claim 7, **characterized in that** gentamicin sulphate, clindamycin hydrochloride, clindamycin hydrogen phosphate, lincosamine hydrochloride, kanamycin sulphate, amikacin sulphate, tobramycin sulphate, tetracycline hydrochloride, chlorotetracycline hydrochloride, oxytetracycline hydrochloride, demethylchlorotetracycline hydrochloride, methacycline hydrochloride, doxycycline hydrochloride, rolitetracycline hydrochloride, minocycline hydrochloride and/or ciprofloxacin hydrochloride and/or moxifloxacin hydrochloride are used as freely water-soluble antibiotic component(s).

9. Process according to any of Claims 1 to 8, **characterized in that** 0.1 to 20.0 percent by mass of the dodecyl sulphate or dodecyl sulphonate, based on the solvent, are used.

10. Process according to any of Claims 1 to 9, **characterized in that** nonwovens, felts, knitted fabrics or continuously knitted fabrics of polyester, collagen or gelatine are antibiotically coated.

11. Process according to any of Claims 1 to 9, **characterized in that** porous mouldings of polyesters, calcium carbonate, calcium sulphate, tricalcium phosphate, hydroxylapatite or resorbable glass are coated.

12. Process according to any of Claims 1 to 9, **characterized in that** metallic mouldings of titanium, titanium alloys or stainless steel are coated.

13. Process for the antibiotic coating of bodies with interconnecting microcavities, **characterized in that** solution containing one or more of the substances from the group consisting of ciprofloxacin dodecylbenzylsulphonate and/or moxifloxacin dodecylsulphate and/or moxifloxacin dodecylbenzylsulphonate and/or moxifloxacin dodecylsulphonate and/or of the dodecylsulphates and/or of the dodecylsulphonates of clindomycin, of tetracycline, of lincosamine, of chlorotetracycline, of oxytetracycline, of demethylchlorotetracycline, of methacycline, of doxycycline, of rolitetracycline and of minocycline is introduced into the microcavities and, after vaporization or evaporation of the solvent, a layer of these substances forms on the surface of the microcavities - the solvent used being at least one organic solvent.

14. Process according to any of Claims 1 to 13, **characterized in that** the microcavities are in the form of pores.

15. Body with interconnecting microcavities, **characterized in that** a layer which comprises gentamicin dodecylsulphate having 1 to 5 dodecylsulphate groups per gentamicin molecule or gentamicin dodecylsulphonate having 1 to 5 dodecylsulphonate groups per gentamicin molecule is formed on the surface of the microcavities.

16. Body according to Claim 15, **characterized in that** the layer comprises gentamicin pentakisdodecylsulphate and/or gentamicin tetrakisdodecylsulphate or gentamicin pentakisdodecylsulphonate and/or gentamicin tetrakisdodecylsulphonate.

17. Body with interconnecting microcavities, **characterized in that** a layer which comprises one or more of the substances from the group consisting of ciprofloxacin dodecylbenzylsulphonate and/or of the dodecylsulphates and/or of the dodecylsulphonates of clindamycin, of tetracycline, of lincosamine, of chlorotetracycline, of oxytetracycline of demethylchlorotetracycline, of methacycline, of doxycycline, of rolitetracycline and minocycline is formed on the surface of the microcavities.

18. Body according to Claim 15 or 17, **characterized in that** it is formed from collagen, gelatin or polyester, calcium carbonate, calcium sulphate, tricalcium phosphate or hydroxylapatite.

19. Body according to Claim 15 or 17, **characterized in that** it is in the form of a nonwoven, felt, continuously knitted fabric or knitted fabric of polyester, collagen or gelatine.

20. Body according to Claim 15 or 17, **characterized in that** it is in the form of a porous moulding of polyester, calcium carbonate, calcium sulphate, tricalcium phosphate, hydroxylapatite or resorbable glass.

21. Body according to Claim 15 or 17, **characterized in that** it is formed from titanium, titanium alloys or stainless steel.

22. Body according to Claim 15 or 17, **characterized in that** the microcavities are in the form of pores.

23. Use of bodies with interconnecting microcavities according to any of Claims 15 to 22 for the production of implants.

## Revendications

1. Procédé de revêtement antibiotique de corps comportant des microcavités interconnectées, **caractérisé en ce qu'**une solution contenant du dodécylsulfate de gentamicine avec 1 à 5 groupes dodécylsulfate par molécule de gentamicine et/ou du dodécylsulfonate de gentamicine avec 1 à 5 groupes dodécylsulfonate par molécule de gentamicine est introduite dans les microcavités et, après vaporisation ou évaporation du solvant, une couche de dodécylsulfate de gentamicine ou de dodécylsulfonate de gentamicine se forme sur la surface des microcavités, le solvant utilisé étant au moins un solvant organique.

2. Procédé selon la revendication 1, **caractérisé en ce que** l'on utilise du pentakis-dodécylsulfate de gentamicine et/ou du tétrakis-dodécylsulfate de gentamicine et/ou du pentakis-dodécylsulfonate de gentamicine et/ou du tétrakis-dodécylsulfonate de gentamicine.

3. Procédé selon la revendication 1, **caractérisé en ce que** l'on utilise un des solvants méthanol, éthanol, N,N-diméthylformamide et diméthylsulfoxyde.

4. Procédé selon l'une des revendications 1 à 3, **caractérisé en ce que** l'introduction se fait par immersion, par pulvérisation ou par dépôt goutte à goutte de la solution.

5. Procédé selon l'une des revendications 1 à 4, **caractérisé en ce que** les corps ont une structure en collagène, gélatine ou polyester, carbonate de calcium, sulfate de calcium, phosphate tricalcique ou hydroxyapatite.

6. Procédé selon l'une des revendications 1 à 5, **caractérisé en ce qu'**une ou plusieurs des substances du groupe comprenant le dodécylbenzylsulfonate de ciprofloxacine et/ou les dodécylsulfates et/ou dodécylsulfonates de la clindamycine, de la tétracycline, de la lincosamine, de la chlortétracycline, de l'oxytétracycline, de la déméthylchlortétracycline, de la méthacycline, de la doxycycline, de la rolitétracycline et de la minocycline sont incorporées en plus dans la solution.

7. Procédé selon l'une des revendications 1 à 6, **caractérisé en ce que**, en premier lieu, une solution aqueuse contenant au moins un composant antibiotique facilement soluble dans l'eau, choisi dans le groupe comprenant les antibiotiques de type aminoglycoside, les antibiotiques de type tétracycline, les antibiotiques de type lincosamide et les antibiotiques de type 4-quinolone, et ensuite, après vaporisation et/ou évaporation de l'eau, une solution de dodécylsulfate de gentamicine ou de dodécylsulfonate de gentamicine, en particulier de pentakis-dodécylsulfate de gentamicine et/ou de tétrakis-dodécylsulfate de gentamicine et/ou de pentakis-dodécylsulfonate de gentamicine et/ou de tétrakis-dodécylsulfonate de gentamicine dans du méthanol, de l'éthanol, du N,N-diméthylformamide et/ou du diméthylsulfoxyde est introduite dans les microcavités interconnectées par immersion ou pulvérisation ou dépôt goutte à goutte.

8. Procédé selon la revendication 7, **caractérisé en ce que** les ou les composants antibiotiques facilement solubles dans l'eau utilisés sont le sulfate de gentamicine, le chlorhydrate de clindamycine, le dihydrogénophosphate de clindamycine, le chlorhydrate de lincosamine, le sulfate de kanamycine, le sulfate d'amikacine, le sulfate de tobramycine, le chlorhydrate de tétracycline, le chlorhydrate de chlortétracycline, le chlorhydrate d'oxytétracycline, le chlorhydrate de déméthylchlortétracycline, le chlorhydrate de méthacycline, le chlorhydrate de doxycycline, le chlorhydrate de rolitétracycline, le chlorhydrate de minocycline et/ou le chlorhydrate de ciprofloxacine et/ou le chlorhydrate de moxifloxacine.

9. Procédé selon l'une des revendications 1 à 8, **caractérisé en ce que** l'on utilise de 0,1 à 20,0% de dodécylsulfate ou dodécylsulfonate, ramené à la masse du solvant.

10. Procédé selon l'une des revendications 1 à 9, **caractérisé en ce que** des non-tissés, des feutres, des tulles ou des tricots en polyester, en collagène ou en gélatine reçoivent un revêtement antibiotique.

11. Procédé selon l'une des revendications 1 à 9, **caractérisé en ce que** des articles façonnés poreux en polyesters, carbonate de calcium, sulfate de calcium, phosphate tricalcique, hydroxyapatite ou verre résorbable sont revêtus.

12. Procédé selon l'une des revendications 1 à 9, **caractérisé en ce que** des articles façonnés métalliques en titane, en alliages de titane ou en acier spécial sont revêtus.

13. Procédé de revêtement antibiotique de corps comportant des microcavités interconnectées, **caractérisé en ce qu'**une solution contenant une ou plusieurs des substances du groupe comprenant le dodécylbenzylsulfonate de ciprofloxacine et/ou le dodécylsulfate de moxifloxacine et/ou le dodécylbenzylsulfonate de moxifloxacine et/ou le dodécylsulfonate de moxifloxacine et/ou les dodécylsulfates et/ou dodécylsulfonates de la clindamycine, de la tétracycline, de la lincosamine, de la chlortétracycline, de l'oxytétracycline, de la déméthylchlortétracycline, de la méthacycline, de la doxycycline, de la rolitétracycline et de la minocycline est introduite dans les microcavités et, après vaporisation ou évaporation du solvant, une couche de ces substances se forme sur la surface des microcavités, le solvant utilisé étant au moins un solvant organique.

14. Procédé selon l'une des revendications 1 à 13, **caractérisé en ce que** les microcavités sont réalisées sous la forme de pores.

15. Corps comportant des microcavités interconnectées, **caractérisé en ce qu'**une couche qui comprend du dodécylsulfate de gentamicine avec 1 à 5 groupes dodécylsulfate par molécule de gentamicine ou du dodécylsulfonate de gentamicine avec 1 à 5 groupes dodécylsulfonate par molécule de gentamicine est formée sur la surface des microcavités.

16. Corps selon la revendication 15, **caractérisé en ce que** la couche comprend du pentakis-dodécylsulfate de gentamicine et/ou du tétrakis-dodécylsulfate de gentamicine ou du pentakis-dodécylsulfonate de gentamicine et/ou du tétrakis-dodécylsulfonate de gentamicine.

17. Corps comportant des microcavités interconnectées, **caractérisé en ce qu'**une couche qui comprend une ou plusieurs des substances du groupe comprenant le dodécylbenzylsulfonate de ciprofloxacine et/ou les dodécylsulfates et/ou dodécylsulfonates de la clindamycine, de la tétracycline, de la lincosamine, de la chlortétracycline, de l'oxytétracycline, de la déméthylchlortétracycline, de la méthacycline, de la doxycycline, de la rolitétracycline et de la minocycline est formée sur la surface des microcavités.

18. Corps selon la revendication 15 ou la revendication 17, **caractérisé en ce qu'**il est constitué de collagène, de gélatine ou de polyester, de carbonate de calcium, de sulfate de calcium, de phosphate tricalcique ou d'hydroxyapatite.

19. Corps selon la revendication 15 ou la revendication 17, **caractérisé en ce qu'**il est constitué d'un non-tissé, d'un feutre, d'un tulle ou d'un tricot en polyester, en collagène ou en gélatine.

20. Corps selon la revendication 15 ou la revendication 17, **caractérisé en ce qu'**il est réalisé sous la forme d'un corps façonné poreux en polyester, carbonate de calcium, sulfate de calcium, phosphate tricalcique, hydroxyapatite ou verre résorbable.

21. Corps selon la revendication 15 ou la revendication 17, **caractérisé en ce qu'**il est constitué de titane, d'alliages de titane ou d'acier spécial.

22. Corps selon la revendication 15 ou 17, **caractérisé en ce que** les microcavités sont réalisées sous la forme de pores.

23. Utilisation de corps comportant des microcavités interconnectées selon l'une des revendications 15 à 22 pour fabriquer des implants.
